# EUROPÄISCHE PATENTANMELDUNG

(11) **EP 4 180 103 A1**
(43) Veröffentlichungstag der Anmeldung: **17.05.2023**
(21) Anmeldenummer: 22205006.4
(22) Anmeldetag: 02.11.2022
(51) Int. Cl.: B01D 3/40, C07C 7/08, C10G 7/08

(54) **VERFAHREN ZUR ENTLEERUNG EINES LÖSUNGSMITTEL-REGENERIERUNGS-BEHÄLTERS SOWIE VORRICHTUNG**

(30) Priorität: 12.11.2021 DE 102021212777; 12.11.2021 BE 202105878
(71) Anmelder: Thyssenkrupp Uhde Engineering Services GmbH, 65812 Bad Soden am Taunus (DE); thyssenkrupp AG, 45143 Essen (DE)
(72) Erfinder: Klukowski, Dirk, 65812 Bad Soden am Taunus (DE); Schuch, Frank, 44229 Dortmund (DE)
(74) Vertreter: thyssenkrupp Intellectual Property GmbH

(57) **Zusammenfassung**

Die vorliegende Erfindung betrifft eine Vorrichtung zur Abtrennung von Aromaten mittels Extraktivdestillation, wobei die Vorrichtung eine Extraktivdestillationskolonne, eine nachgeschaltete Abtreiberkolonne sowie einen Lösungsmittel-Regenerierungs-Behälter 10 aufweist, dadurch gekennzeichnet, dass der Lösungsmittel-Regenerierungs-Behälter 10 an der Unterseite ein erstes Ventil 20 aufweist, wobei das erste Ventil 20 auf der dem Lösungsmittel-Regenerierungs-Behälter 10 abgewandten Seite mit einer beheizten Leitung 30 verbunden ist, wobei die beheizte Leitung 30 mit einer Anlage 40 verbunden ist ausgewählt aus der Liste umfassend Vakuumdestillationsanlage, Rohöldestillationsanlage, Verbrennungsanlage, Lagereinrichtung, Verladeeinrichtung, Raffinerie.

## Beschreibung

Die Erfindung betrifft ein Verfahren zur Entleerung eines Lösungsmittel-Regenerierungs-Behälters sowie eine Vorrichtung hierfür.

Aromaten, insbesondere die einfachsten aromatischen Verbindungen Benzol, Toluol und Xylol, sind von großtechnischer Bedeutung als Zwischenprodukte für die chemische Industrie. Für die Gewinnung von Aromaten sind verschiedene technische Verfahren bekannt. Eine Verfahrensart, welche bei vergleichsweise geringen Kosten eine besonders hohe Reinheit des Aromaten-Produktstroms erreicht, ist die Gewinnung durch Extraktivdestillation aus einem kohlenwasserstoffhaltigen Einsatzstoffstrom. Als Einsatzstoffstrom kommen beispielsweise Rohbenzin, Pyrolysebenzin, Reformatbenzin oder auch Kokerei-Leichtöl in Betracht. Schwere Komponenten werden bevorzugt vor der Aromatengewinnung, beispielsweise durch Abtrennung der C8+-Fraktion, aus dem Einsatzstoffstrom entfernt.

Bei der Gewinnung von Aromaten durch Extraktivdestillation wird der Einsatzstoffstrom mit einem selektiven Lösungsmittel für Aromaten im Gegenstrom in Kontakt gebracht. Das Lösungsmittel beeinflusst die Flüchtigkeit der unterschiedlichen Bestandteile des Einsatzstoffstroms unterschiedlich stark. Die Flüchtigkeit der aromatischen Bestandteile wird durch die Verdünnung herabgesetzt, während die der aliphatischen Bestandteile deutlich erhöht wird. Auf diese Weise wird eine destillative Auftrennung in Aromaten und Nichtaromaten/Aliphaten ermöglicht. In einem ersten Schritt werden die im Lösungsmittel gelösten Aromaten dann mittels extraktiver Destillation von den aliphatischen Bestandteilen der Einsatzmischung getrennt, welche als Kopfprodukt der Destillation abgeführt werden. In einem zweiten Schritt werden die Aromaten durch Strippung als Kopfprodukt aus dem Lösungsmittel getrieben. Anschließend kann der Aromaten-Produktstrom in einzelne Aromatenfraktionen weiter aufgetrennt werden. Das an Aromaten abgereicherte Lösungsmittel wird in die extraktive Destillation zurückgeführt und wiederverwendet. Auf diese Weise lassen sich in einem kontinuierlichen Prozess einzelne Aromaten in Reinform gewinnen.

Als Lösungsmittel für die Extraktivdestillation sind unter anderem Sulfolan, Methylsulfolane, N-Methylpyrrolidon, N-Formylmorpholin, Ethylenglykol und deren Mischungen, sowie Mischungen aus den genannten Lösungsmitteln mit Wasser bekannt. Die verwendeten Lösungsmittel bzw. Lösungsmittelmischungen sind wasserlöslich.

Aus der EP 154 677 A2 ist ein Verfahren zur Abtrennung von Aromaten aus Kohlenwasserstoffgemischen beliebigen Aromatengehaltes bekannt.

Aufgrund der dauerhaften Wiederverwendung desselben Lösungsmittels in einem Lösungsmittelkreislauf sammeln sich Verunreinigungen, welche den Prozess weder als Kopfprodukt der destillativen Abtrennung der Aliphaten noch als Kopfprodukt bei der Strippung der Aromaten verlassen, im Lösungsmittel an. Weiterhin können sich durch der Extraktivdestillation vorgeschaltete Prozesse, wie beispielsweise Hydrierung oder Clay-Treatment, schwere Komponenten eingebracht werden, die sich im Lösemittel anreichern. Aber auch aufgrund von Fehlbedienung oder Fehlfunktionen von der Extraktivdestillation vorgeschalteten Anlagenteilen, die beispielsweise der Entfernung höher siedender Anteile dienen, kann es zu einer erhöhten Kontamination des in der extraktiven Destillation umlaufenden Lösemittels mit schwersiedenden Verunreinigungen kommen.

Die Verunreinigungen führen im Laufe der Zeit dazu, dass das Lösungsmittel seine Extraktionskraft verliert und ausgetauscht werden muss. Um die mit dem Austausch des Lösungsmittels verbundenen Kosten zu reduzieren, ist die Möglichkeit einer Aufreinigung des Lösungsmittels im Lösungsmittelkreislauf wünschenswert, durch die die Verunreinigungen entfernt werden. Auf diese Weise kann die Zyklusdauer bis zum Austausch des Lösungsmittels erheblich verlängert werden.

Zur Aufreinigung des Lösungsmittels ist es beispielsweise aus der US 2010/0228072 A1 bekannt, einen Teilstrom des Lösungsmittelkreislaufes einer Destillation zu unterziehen, wobei das aufgereinigte Lösungsmittel die Destillation als Kopfprodukt verlässt und als Destillationsrückstand zurückbleibende Verunreinigungen aus der Anlage entfernt werden.

Bei der Lösungsmittelregenerierung wird ein Teilstrom des Lösungsmittels in einer Blase Überkopf destilliert (batch oder kontinuierlich) und es verbleiben Komponenten, die im Vergleich zum Lösungsmittel einen höheren Siedepunkt haben. Dieses sind beispielsweise Polymere, Rost und Lösungsmittelreste. Dieser hochviskose Rückstand verbleibt im Lösungsmittel-Regenerierungs-Behälter ein hochviskoser Rückstand aus Lösungsmittelresten, sich in der Anlage gebildeten längerkettigen Kohlenwasserstoffen, Rostpartikel und ähnlichem zurück. Dieser wird heutzutage in Fässer abgefüllt und anschließend beispielsweise der Verbrennung zugeführt. Um eine Abfüllung in Fässer zu ermöglichen, ist eine erhöhte Temperatur von üblicherweise 160 °C notwendig. Dieses wiederum bedeutet neben dem logistischen Aufwand aber auch, dass insbesondere durch Spritzer beim Abfüllprozess eine erhöhte Gefahr ausgeht, weshalb entsprechende Sicherheitsvorschriften und -Installationen notwendig sind.

Aus der CN 203976565 U ist eine Vorrichtung zur Wiederverwertung von flüssigen Lycopinabfällen bekannt.

Aufgabe der Erfindung ist es, ein Verfahren zur Entsorgung der hochsiedenden Rückstände und eine dafür geeignete Vorrichtung bereitzustellen, welches einfacher und sicherer ist.

Gelöst wird diese Aufgabe durch die Vorrichtung mit den in Anspruch 1 angegebenen Merkmalen sowie durch das Verfahren mit den in Anspruch 7 abgegebenen Merkmalen. Vorteilhafte Weiterbildungen ergeben sich aus den Unteransprüchen, der nachfolgenden Beschreibung sowie der Zeichnung.

Die erfindungsgemäße Vorrichtung zur Abtrennung von Aromaten mittels Extraktivdestillation weist eine Extraktivdestillationskolonne, eine nachgeschaltete Abtreiberkolonne sowie einen Lösungsmittel-Regenerierungs-Behälter auf. Entsprechende Vorrichtungen zur Abtrennung von Aromaten mittels Extraktivdestillation sind beispielsweise aus der DE 19849651 A1, DE 19958464 A1, DE 10019196 C1, DE 10018434 A1 oder DE 10038318 C1 sowie unter dem Begriff Morphylane-Verfahren bekannt.

Erfindungsgemäß weist der Lösungsmittel-Regenerierungs-Behälter an der Unterseite ein erstes Ventil auf. Das erste Ventil ist auf der dem Lösungsmittel-Regenerierungs-Behälter abgewandten Seite mit einer beheizten Leitung verbunden. Die beheizte Leitung ist mit einer Anlage verbunden ausgewählt aus der Liste umfassend Vakuumdestillationsanlage, Rohöldestillationsanlage, Verbrennungsanlage, Lagereinrichtung, Verladeeinrichtung, Raffinerie.

Während bisher der sich in dem Lösungsmittel-Regenerierungs-Behälter ansammelnde Rückstand in Fässer abgefüllt und entsorgt wurde, kann nun dieser Rückstand ohne direkte menschliche Interaktion einer Weiterverarbeitung zugeführt werden. Hierdurch kann zum einen bereits der Lösungsmittel-Regenerierungs-Behälter niedriger angeordnet werden, üblicherweise etwa 2 m, da unter dem Lösungsmittel-Regenerierungs-Behälter kein Platz für die Anordnung von Fässern vorgesehen werden muss. Hierdurch wird zum einen Aufwand bei der Erstellung der Vorrichtung reduziert. Zum anderen wird durch die niedrigere Anordnung Flexibilität auch für andere Anlagenteile gewonnen.

Zwar erscheint es zunächst nicht notwendig, eine feste Verrohrung durchzuführen, zumal dadurch zum einen Lösungsmittel und zum anderen Heizenergie verbraucht wird und der Rückstand üblicherweise nur selten aus dem Lösungsmittel-Regenerierungs-Behälter entfernt werden muss. Der Gewinn liegt jedoch darin, dass auf das Handling der Fässer und damit die direkte Einbindung von Menschen in diesen Prozess mit einer heißen, hochviskosen Flüssigkeit vermieden werden kann und so die Sicherheit der Vorrichtung erhöht werden kann. Zusätzlich hat sich gezeigt, dass die für diesen Prozess notwendigen aromatischen Lösungsmittel und nicht-aromatischen Lösungsmittel wenigstens zu einem großen Teil zurückgewonnen werden können.

In einer weiteren Ausführungsform der Erfindung ist die beheizte Leitung mit einer Zuführung für nicht-aromatische Lösungsmittel verbunden. Somit kann die beheizte Leitung mit dem nicht-aromatischen Lösungsmittel gespült werden. Die Verwendung von nicht-aromatischem Lösungsmittel ist bevorzugt, um das wertvollere aromatische Lösungsmittel zu sparen. Zusätzlich ist eine Einleitung in eine Vakuumdestillationsanlage oder eine Rohöldestillationsanlage bevorzugt, sodass das nicht-aromatische Lösungsmittel leicht wieder zurückgewonnen werden kann.

In einer weiteren Ausführungsform der Erfindung ist der Lösungsmittel-Regenerierungs-Behälter mit einer Zuführung für aromatische Lösungsmittel verbunden. Der Lösungsmittel-Regenerierungs-Behälter ist im den Kreislauf zur Gewinnung von Aromaten eingebunden, sodass man die im Gesamtprozess gewonnenen Aromaten verunreinigen würde, wenn man den Lösungsmittel-Regenerierungs-Behälter mit nicht-aromatischen Lösungsmitteln auswäscht. Bevorzugt stammen die aromatischen Lösungsmittel aus dem Gesamtprozess und werden durch die bevorzugte Einleitung in eine Vakuumdestillationsanlage oder eine Rohöldestillationsanlage der erneuten Gewinnung wieder zugeführt.

In einer weiteren Ausführungsform der Erfindung ist der Lösungsmittel-Regenerierungs-Behälter mit einer Gaszuführung verbunden. Besonders bevorzugt dient die Gaszuführung zur Beaufschlagung mit Stickstoff. Bevorzugt dient die Gaszuführung zur Beaufschlagung mit einem Druck von 1,5 bar bis 5 bar, bevorzugt mit 2 bar bis 3 bar. Hierdurch kann der hochviskose Rückstand einfacher aus dem Lösungsmittel-Regenerierungs-Behälter durch die beheizte Leitung abgeführt werden.

In einer weiteren Ausführungsform der Erfindung weist die beheizte Leitung eine Länge von 100 m bis 2000 m, bevorzugt von 500 m bis 1200 m, auf. Wird die beheizte Leitung länger, so steigt der Druckverlust über die beheizte Leitung zu stark an. Daher wäre dann die beheizte Leitung stärker zu Erwärmen um die Viskosität zu senken, was wiederum die Betriebskosten deutlich steigern würde.

In einer weiteren Ausführungsform der Erfindung ist die Anlage eine Vakuumdestillationsanlage oder eine Rohöldestillationsanlage. Hierdurch ist eine optimale Rückgewinnung der aromatischen Lösungsmittel und der nicht-aromatischen Lösungsmittel möglich.

In einem weiteren Aspekt betrifft die Erfindung ein Verfahren zur Entleerung einer erfindungsgemäßen Vorrichtung. Das Verfahren weist die folgenden Schritte auf:
a) Zuführen von aromatischem Lösungsmittel in den Lösungsmittel-Regenerierungs-Behälter,
b) Öffnen des ersten Ventils,
c) Überführen des im aromatischen Lösungsmittel gelösten Rückstands durch die beheizte Leitung in eine Anlage ausgewählt aus der Liste umfassend Vakuumdestillationsanlage, Rohöldestillationsanlage, Verbrennungsanlage, Lagereinrichtung, Verladeeinrichtung, Raffinerie.

Durch das Zuführen eines aromatischen Lösungsmittels wird der Rückstand verdünnt, dessen Viskosität gesenkt. Gleichzeitig wird eine Kontamination mit nicht-aromatischen Lösungsmitteln vermieden, welche sich sonst im aromatischen Produkt wiederfinden würden.

Da das erste Ventil an der Unterseite des Lösungsmittel-Regenerierungs-Behälters angeordnet ist, kann durch dessen Öffnen des ersten Ventils in Schritt b) der mit aromatischem Lösungsmitteln verdünnte Rückstand in die beheizte Leitung in Schritt c) überführt werden. Dieses kann alleine durch die Schwerkraft oder durch zusätzlichen Druck erzielt werden.

Dadurch, dass der Rückstand somit direkt durch die beheizte Leitung entfernt und nicht in Fässer umgefüllt werden muss, ist es ausgeschlossen, dass heißes Material als Spritzer austreten und umstehendes Personal verletzen kann.

In einer weiteren Ausführungsform der Erfindung wird in Schritt a) aus in der Vorrichtung zur Abtrennung von Aromaten mittels Extraktivdestillation gewonnenes aromatische Lösungsmittel verwendet.

In einer weiteren Ausführungsform der Erfindung wird in Schritt a) eine Viskosität von 5 cP bis 500 cP, bevorzugt von 10 cP bis 100 cP, besonders bevorzugt von 20 cP bis 40 cP, bei 80 °C eingestellt (1 cP = 10⁻³ Pa · s). Die Viskosität wird mit einem Viskosimeter gemäß EN ISO 3219 ermittelt. Bei dieser Viskosität ist ein Transport der Rückstände über eine sinnvolle Entfernung in einem chemischen Anlagenverbund sinnvoll möglich, ohne dass zu hohe Druckverluste über die Gesamtstrecke auftreten.

In einer weiteren Ausführungsform der Erfindung erfolgt das Überführen in Schritt c) durch Beaufschlagen des Lösungsmittel-Regenerierungs-Behälters durch Beaufschlagung mit einem Gas, vorzugsweise Stickstoff. Besonders bevorzugt erfolgt das Beaufschlagen mit einem Druck von 1,5 bar bis 5 bar, weiter bevorzugt mit 2 bar bis 3 bar.

In einer weiteren Ausführungsform der Erfindung werden nach dem Schritt c) die folgenden Schritte durchgeführt:
d) Schließen des ersten Ventils,
e) Spülen der beheizten Leitung mit einem nicht-aromatische Lösungsmittel.

Für das Spülen in Schritt e) bevorzugt ein zweites Ventil geöffnet, welches die Zuführung für nicht-aromatische Lösungsmittel mit der beheizten Leitung verbindet. Hierdurch kann kein Rückstand aus dem Lösungsmittel-Regenerierungs-Behälter in die Zuführung für nicht-aromatische Lösungsmittel gelangen.

In einer weiteren Ausführungsform der Erfindung erfolgt das Zuführen in Schritt a) derart, dass das Volumen des zugeführten aromatischen Lösungsmittels etwa dem einfachen bis dem zweifaches des Volumens des Rückstandes entspricht. Hierdurch kann üblicherweise eine Verdünnung erzielt werden, welche die benötigte Viskosität der Lösung einstellt.

In einer weiteren Ausführungsform der Erfindung wird die beheizte Leitung auf eine Temperatur von 40 °C bis 100 °C, bevorzugt von 50 °C bis 80 °C, beheizt. Dieses stellt ein Optimum zwischen hoher Temperatur zur Erniedrigung der Viskosität und damit des Druckverlustes sowie niedriger Temperatur zur Reduzierung der Kosten für das Erwärmen dar. Temperaturen unter 100 °C sind bevorzugt um ein Verdampfen des nicht-aromatischen Lösungsmittel zu vermeiden oder wenigstens zu minimieren.

In einer weiteren Ausführungsform der Erfindung wird in Schritt a) als aromatische Lösungsmittel Benzol, Toluol, Xylol oder eine Mischung hieraus verwendet.

In einer weiteren Ausführungsform der Erfindung wird vor Schritt a) der Lösungsmittel-Regenerierungs-Behälter auf etwa 160 °C beheizt und wird durch Zuführung des aromatischen Lösungsmittels in Schritt a) auf etwa 100 °C abgekühlt.

Nachfolgend ist die erfindungsgemäße Vorrichtung anhand eines in der Zeichnung dargestellten Ausführungsbeispiels näher erläutert.

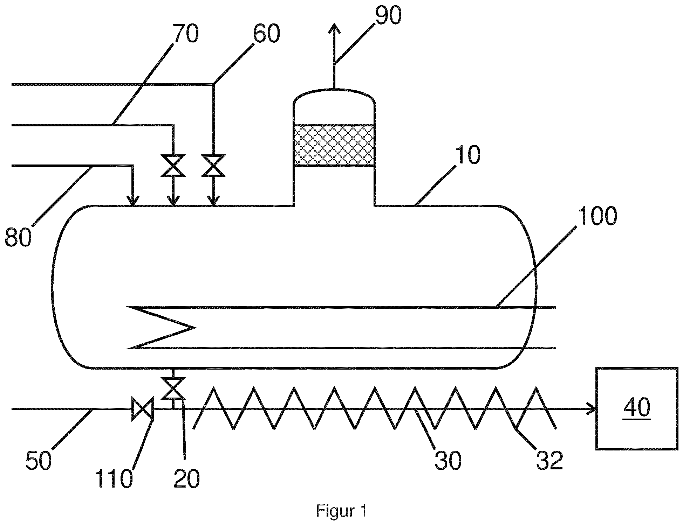

### Fig. 1 schematische Darstellung des Lösungsmittel-Regenerierungs-Behälters

Die Darstellung ist rein schematisch und dient zur Veranschaulichung der Erfindung. Insbesondere ist die Darstellung nicht maßstabsgerecht, was schon aus den linienförmig dargestellten Leitungen erkennbar ist.

In Fig. 1 ist der erfindungsgemäße Lösungsmittel-Regenerierungs-Behälter 10 anhand einer Skizze verdeutlicht. Im laufenden Betrieb wird über die Extraktionslösungsmittelzuführung 80 Extraktionslösungsmittel, beispielsweise Sulfolan, Methylsulfolane, N-Methylpyrrolidon, N-Formylmorpholin, Ethylenglykol und deren Mischungen, sowie Mischungen aus den genannten Lösungsmitteln mit Wasser, zugeführt und durch die Erwärmung mittels der Heizung 100 durch die Extraktionslösungsmittelabführung 90 entnommen. Es reichert sich hierbei ein Rückstand im Lösungsmittel-Regenerierungs-Behälter 10 an, welcher in größeren Abständen entfernt werden muss. Hierzu wird die Zufuhr an Extraktionslösungsmittel beendet. Über die Zuführung für aromatische Lösungsmittel 60 wird aromatisches Lösungsmittel, beispielsweise Benzol, Toluol, Xylol oder deren Mischungen, zugeführt, sodass sich eine Viskosität der Lösung im Inneren des Lösungsmittel-Regenerierungs-Behälters 10 von etwa 30 cP und eine Temperatur von 100 °C einstellt. Dann wird das erste Ventil 20 geöffnet und über die Gaszuführung 70 Stickstoff mit einem Druck von 2,5 bar aufgegeben. Hierdurch wird der gelöste Rückstand durch die beheizte Leitung 30 zur Anlage 40, beispielsweise einer Vakuumdestillationsanlage, zugeführt. Die beheizte Leitung 30 wird hierbei mittels der Heizung der beheizten Leitung 32 auf beispielsweise 80 °C geheizt. Nach Entleerung des Lösungsmittel-Regenerierungs-Behälters 10 wird das erste Ventil 20 geschlossen. Anschließend wird das zweite Ventil 110 geöffnet und die beheizte Leitung 30 mit einem nicht aromatischen Lösungsmittel aus der Zuführung für nicht-aromatische Lösungsmittel 50 gespült. Anschließend wird das zweite Ventil 110 wieder geschlossen.

Die vier in der Fig. 1 gezeigten Ventile können manuell betätigt werden. Alternativ und bevorzugt handelt es sich um Regelventile, also um Ventile, die durch ein zentrales Steuersystem elektronisch geregelt, insbeondere geöffnet und geschlossen werden können.

### Bezugszeichen

- 10: Lösungsmittel-Regenerierungs-Behälter
- 20: erstes Ventil
- 30: beheizte Leitung
- 32: Heizung der beheizten Leitung
- 40: Anlage
- 50: Zuführung für nicht-aromatische Lösungsmittel
- 60: Zuführung für aromatische Lösungsmittel
- 70: Gaszuführung
- 80: Extraktionslösungsmittelzuführung
- 90: Extraktionslösungsmittelabführung
- 100: Heizung
- 110: zweites Ventil

## Patentansprüche

1. Vorrichtung zur Abtrennung von Aromaten mittels Extraktivdestillation, wobei die Vorrichtung eine Extraktivdestillationskolonne, eine nachgeschaltete Abtreiberkolonne sowie einen Lösungsmittel-Regenerierungs-Behälter (10) aufweist, **dadurch gekennzeichnet, dass** der Lösungsmittel-Regenerierungs-Behälter (10) an der Unterseite ein erstes Ventil (20) aufweist, wobei das erste Ventil (20) auf der dem Lösungsmittel-Regenerierungs-Behälter (10) abgewandten Seite mit einer beheizten Leitung (30) verbunden ist, wobei die beheizte Leitung (30) mit einer Anlage (40) verbunden ist ausgewählt aus der Liste umfassend Vakuumdestillationsanlage, Rohöldestillationsanlage, Verbrennungsanlage, Lagereinrichtung, Verladeeinrichtung, Raffinerie.

2. Vorrichtung nach Anspruch 1, **dadurch gekennzeichnet, dass** die beheizte Leitung (30) mit einer Zuführung für nicht-aromatische Lösungsmittel (50) verbunden ist.

3. Vorrichtung nach einem der vorstehenden Ansprüche, **dadurch gekennzeichnet, dass** der Lösungsmittel-Regenerierungs-Behälter (10) mit einer Zuführung für aromatische Lösemittel (60) verbunden ist.

4. Vorrichtung nach einem der vorstehenden Ansprüche, **dadurch gekennzeichnet, dass** der Lösungsmittel-Regenerierungs-Behälter (10) mit einer Gaszuführung (70) verbunden ist.

5. Vorrichtung nach einem der vorstehenden Ansprüche, **dadurch gekennzeichnet, dass** die beheizte Leitung (30) eine Länge von 100 m bis 2000 m, bevorzugt von 500 m bis 1200 m, aufweist.

6. Vorrichtung nach einem der vorstehenden Ansprüche, **dadurch gekennzeichnet, dass** die Anlage (40) eine Vakuumdestillationsanlage oder eine Rohöldestillationsanlage ist.

7. Verfahren zur Entleerung einer Vorrichtung nach einem der vorstehenden Ansprüche, wobei das Verfahren die folgenden Schritte aufweist:
a) Zuführen von aromatischem Lösungsmittel in den Lösungsmittel-Regenerierungs-Behälter (10),
b) Öffnen des ersten Ventils (20),
c) Überführen des im aromatischen Lösungsmittel gelösten Rückstands durch die beheizte Leitung (30) in eine Anlage (40) ausgewählt aus der Liste umfassend Vakuumdestillationsanlage, Rohöldestillationsanlage, Verbrennungsanlage, Lagereinrichtung, Verladeeinrichtung, Raffinerie.

8. Verfahren nach Anspruch 7, **dadurch gekennzeichnet, dass** in Schritt a) aus in der Vorrichtung zur Abtrennung von Aromaten mittels Extraktivdestillation gewonnenes aromatische Lösungsmittel verwendet wird.

9. Verfahren nach einer der Ansprüche 7 bis 8, **dadurch gekennzeichnet, dass** in Schritt a) eine Viskosität von 5 cP bis 500 cP, bevorzugt von 10 cP bis 100 cP, besonders bevorzugt von 20 cP bis 40 cP, bei 80 °C eingestellt wird.

10. Verfahren nach einer der Ansprüche 7 bis 9, **dadurch gekennzeichnet, dass** das Überführen in Schritt c) durch Beaufschlagen des Lösungsmittel-Regenerierungs-Behälters (10) durch Beaufschlagung mit einem Gas, vorzugsweise Stickstoff, erfolgt.

11. Verfahren nach Anspruch 10, **dadurch gekennzeichnet, dass** das Beaufschlagen mit einem Druck von 1,5 bar bis 5 bar, bevorzugt mit 2 bar bis 3 bar, erfolgt.

12. Verfahren nach einer der Ansprüche 7 bis 11, **dadurch gekennzeichnet, dass** nach dem Schritt c) die folgenden Schritte durchgeführt werden:
d) Schließen des ersten Ventils (20),
e) Spülen der beheizten Leitung (30) mit einem nicht-aromatische Lösungsmittel.

13. Verfahren nach einer der Ansprüche 7 bis 12, **dadurch gekennzeichnet, dass** das Zuführen in Schritt a) derart erfolgt, dass das Volumen des zugeführten aromatischen Lösungsmittels etwa dem einfachen bis dem zweifaches des Volumens des Rückstandes entspricht.

14. Verfahren nach einer der Ansprüche 7 bis 13, **dadurch gekennzeichnet, dass** die beheizte Leitung (30) auf eine Temperatur von 40 °C bis 100 °C, bevorzugt von 50 °C bis 80 °C, beheizt wird.

15. Verfahren nach einer der Ansprüche 7 bis 14, **dadurch gekennzeichnet, dass** in Schritt a) als aromatische Lösungsmittel Benzol, Toluol, Xylol oder eine Mischung hieraus verwendet wird.

16. Verfahren nach einer der Ansprüche 7 bis 15, **dadurch gekennzeichnet, dass** vor Schritt a) der Lösungsmittel-Regenerierungs-Behälter (10) auf etwa 160 °C beheizt wird und durch Zuführung des aromatischen Lösungsmittels in Schritt a) auf etwa 100 °C abgekühlt wird.
